# EUROPÄISCHE PATENTSCHRIFT

(11) **EP 2 448 602 B1**
(45) Veröffentlichungstag und Bekanntmachung des Hinweises auf die Patenterteilung: **06.03.2013**
(21) Anmeldenummer: 10812856.2
(22) Anmeldetag: 22.12.2010
(51) Int. Cl.: A61L 2/20, B01B 1/00

(54) **BLITZDAMPFERZEUGER SOWIE ANORDNUNG MIT BLITZDAMPFERZEUGER**
FLASH VAPOR GENERATOR AND ASSEMBLY COMPRISING A FLASH VAPOR GENERATOR
GÉNÉRATEUR DE VAPEUR ÉCLAIR ET DISPOSITIF POURVU D'UN GÉNÉRATEUR DE VAPEUR ÉCLAIR

(30) Priorität: 23.12.2009 DE 102009060512
(43) Veröffentlichungstag der Anmeldung: 09.05.2012
(73) Patentinhaber: Metall + Plastic GmbH, 78315 Radolfzell (DE)
(72) Erfinder: VON STENGLIN, Christoph, 78315 Radolfzell (DE)
(74) Vertreter: Wagner, Kilian
(86) Internationale Anmeldenummer: PCT/EP2010/007862
(87) Internationale Veröffentlichungsnummer: WO 2011/076400

(56) Entgegenhaltungen:
- EP-A2- 0 243 003
- EP-A2- 1 738 777
- WO-A1-2006/108796
- WO-A2-98/34649

## Beschreibung

Die Erfindung betrifft einen Vorrichtung zur Erzeugung von Dekontaminationsmitteldampf, insbesondere von Wasserstoffperoxyddampf, umfassend einen Verdampferkörper, eine Heizeinrichtung zum Erhitzen des Verdampferkörpers sowie mehrere Zuführkanäle zum Zuführen von zu verdampfender Dekontaminationsflüssigkeit, vorzugsweise einer wasserstoffperoxydhaltigen Flüssigkeit. Bevorzugt betrifft die Erfindung einen sogenannten Blitzdampferzeuger (Schnelldampferzeuger). Ferner betrifft die Erfindung eine vorzugsweise pharmazietechnische Anordnung, umfassend einen zu dekontaminierenden Raum, insbesondere einen Isolator und/oder eine Schleuse sowie eine Vorrichtung zur Erzeugung von Dekontaminationsmitteldampf.

Zur Dekontamination von Isolatoren und/oder Schleusen wird in der Pharmaindustrie aufgrund seiner hohen Reaktivität Wasserstoffperoxyddampf eingesetzt. Dieser wird durch Verdampfen einer wässrigen Wasserstoffperoxydlösung erhalten. Zur Minimierung einer Explosionsgefahr bei der Verdampfung von wasserstoffperoxydhaltigen Lösungen sind sogenannte Blitzverdampfer (Blitzdampferzeuger) im Einsatz, mit dem Ziel, kontinuierlich kleine Mengen von wasserstoffperoxydhaltiger Flüssigkeit schlagartig (blitzartig) zu verdampfen. Ein Sieden größerer Mengen an wasserstoffperoxydhaltiger Flüssigkeit ist aufgrund der vorgenannten Explosionsgefahr nicht zulässig. Die Schwierigkeit beim Verdampfen kleiner Mengen wasserstoffperoxydhaltiger Flüssigkeit, insbesondere von wässrigen Lösungen ist die Bildung von auf heißer Verdampferfläche "tanzender" Flüssigkeitstropfen, die den Bestrebungen einer blitzartigen Verdampfung zuwiderlaufen.

Aus der DE 10 2006 006 095 A1 ist ein Wasserstoffperoxyddampferzeuger bekannt, der eine ebene Verdampferfläche aufweist. Hier kann es zu der vorerwähnten "tanzenden" Tröpfchenbildung kommen.

Aus der EP 0 972 159 B1 ist ein alternativer Blitzverdampfer (Schnellverdampfer) bekannt, der sich durch hydraulisch miteinander kommunizierend angeordnete Verdampferkanäle in einem Verdampferkörper auszeichnet. Der Aufbau ist vergleichsweise komplex.

Hinsichtlich weiteren Standes der Technik wird auf die DE 602 03 603 T2 oder die DE 603 00 820 T2 verwiesen.

Aus der DE 2005 030 822 A1 ist ein Wasserstoffperoxydverdampfer mit einem topfartigen Gehäuse und einem Verdampferkörper bekannt, der eine einzige, großflächige Verdampferoberfläche aufweist, wobei die Wärmezufuhr in das Dekontaminationsmittel ausschließlich von unten erfolgt. Der bekannte Verdampfer scheint hinsichtlich seiner Verdampfungsrate sowie hinsichtlich der Vermeidung einer "tanzenden" Dekontaminationsmitteldampftröpfchen verbesserungsbedürftig. Aus der DE 2005 030 822 A1 ist es zudem bekannt, mehrere Verdampfer über jeweils eine Leitung an ein zu sterilisierendes Gefäß anzuschließen, um die Dekontaminationsdampfmenge zu erhöhen. Die gesamten Verdampferkosten fallen also x-Mal an. Zudem müssen eine Vielzahl von Dampfleitungen in den zu verdampfenden Raum geführt werden, was bei kleinen Räumen aus Platzgründen problematisch ist. Zudem müssen dann eine Vielzahl von Dichtungen vorgesehen werden.

Aus der CN 2009 43844 Y ist ein Verdampfer für Wasser bekannt. Der bekannte Verdampfer weist einen Verdampfungskörper mit einer Vielzahl kleiner Löcher auf. Diesen ist gemeinsam ein einziger Zulaufkanal zugeordnet, der mittig oberhalb des Verdampferkörpers angeordnet ist. Damit die Vielzahl von kleinen Löchern ihren Beitrag zur Verdampfung leisten können, muss durch den einzigen Zuführkanal eine ausreichend große Flüssigkeitsmenge zugeführt werden, was dann jedoch dem Ziel einer spontanen Blitzverdampfung von Dekontaminationsmitteln zuwider laufen würde. In der Praxis käme es zu einem gefährlichen Verkochen von Dekontaminationsmitteln. Daher ist der bekannte Verdampfer nicht zum Verdampfen von Dekontaminationsmitteln geeignet.

Aus der EP 1738777 ist ein Verdampfer für eine Sterilisationsappartur mit vier Zuführkanälen bekannt, durch die eine Dekontaminationsflüssigkeit auf eine beheizbare Platte gesprüht wird. Die Verdampferoberfläche kann Vertiefungen beispielsweise in der Form von Halbkugeln aufweisen.

Sämtliche vorgenannten Blitzverdampfer zeichnen sich durch einen vergleichsweise komplexen Aufbau und/oder durch eine verbesserungswürdige Verdampfungsrate aus.
Ausgehend von dem vorgenannten Stand der Technik liegt der Erfindung die Aufgabe zugrunde, einen möglichst einfach aufgebauten Blitzverdampfer (Schnellverdampfer) zur Erzeugung von Dekontaminationsmitteldampf anzugeben, der sich durch eine hohe Verdampfungsrate auszeichnet. Bevorzugt soll die Bildung "tanzender" Dekontaminationsmittelflüssigkeitstropfen innerhalb des Verdampfers weitgehend vermieden werden. Darüber hinaus besteht die Aufgabe darin, eine Anordnung, umfassend einen zu dekontaminierenden Raum und einen entsprechend verbessert Dampferzeuger, anzugeben. Bevorzugt handelt es sich um eine Anordnung zur Abfüllung und/oder Verarbeitung von Pharmazeutika.

Hinsichtlich des Schnelldampferzeugers wird die Aufgabe bei einer gattungsgemäßen Vorrichtung dadurch gelöst, dass in dem ein- oder mehrteiligen Verdampferkörper mehrere Sacklöcher vorgesehen sind und dass den Sacklöchern jeweils mindestens einer, vorzugsweise ausschließlich einer, der Zuführkanäle zugeordnet ist, um über diese die in einzelnen Sacklöchern zu verdampfende Dekontaminationsmittelflüssigkeit, vorzugsweise tropfenweise, zuzuführen. Bevorzugt handelt es sich bei der zu verdampfenden Dekontaminationsflüssigkeit um eine wässrige Lösung von Wasserstoffperoxyd, ganz besonders bevorzugt um eine 35%ige bis 50%ige Lösung.

Hinsichtlich der Anordnung, bevorzugt einer pharmatechnischen Anordnung, wird die Aufgabe durch den Einsatz eines wie zuvor beschrieben ausgebildeten Dampferzeugers gelöst.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen. Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale als vorrichtungsgemäß offenbart gelten und beanspruchbar sein.

Der Erfindung liegt die Erkenntnis zugrunde, dass eine Schnellverdampfung von vorzugsweise tropfenförmig zugeführter Dekontaminationsflüssigkeit im Hinblick auf die Verdampfungsrate auf verbesserte und einfache Weise dadurch erreicht werden kann, dass die Verdampfung innerhalb von (umfangsgeschlossenen) Sacklöchern eines ein- oder mehrteiligen Verdampferkörpers stattfindet. Dies ist u.a. darauf zurückzuführen, dass die erhitzte Umfangswand der Sacklöcher einen wesentlich geringeren Abstand zu der zugeführten, zum Verdampfen der Dekontaminationsflüssigkeit aufweist als eine Umfangswand eines zylindrischen Verdampfers mit ebener Bodenfläche. Bei einem nach dem Konzept der Erfindung ausgebildeten Schnellverdampfer kann der zu verdampfenden, vorzugsweise tropfenförmig zugeführten, Dekontaminationsflüssigkeit hierdurch in kürzester Zeit eine größere Wärmemenge zugeführt werden, und zwar nicht nur von unten, sondern auch durch Strahlungswärme von der Umfangswand des entsprechenden Sacklochs. Durch das Vorsehen von in einem Verdampferkörper (insbesondere einem Verdampferblock) vorgesehenen Sacklöchern, die beispielsweise als Bohrungen hergestellt werden können, wird ein äußerst einfacher und effektiver Aufbau der Verdampfungsvorrichtung erzielt. Im Gegensatz zu aus dem Stand der Technik bekannten Verdampfern ist es nicht notwendig, den Verdampferkörper durchziehende Verdampferkanäle bereitzustellen, durch die die zu verdampfende Flüssigkeit geleitet wird, wobei die vorgenannten Kanäle im Stand der Technik auch noch aufwändig hydraulisch miteinander verbunden sind. Ganz besonders zweckmäßig ist eine Ausführungsvariante der Vorrichtung zur Erzeugung von Dekontaminationsmitteldampf, bei der der, bevorzugt einteilige, Verdampferkörper zur Erzielung einer optimalen Wärmeleitung aus Aluminium, insbesondere einer Aluminiumlegierung ausgebildet ist. Noch weiter bevorzugt ist es, wenn die Heizeinrichtung derart ausgebildet bzw. eingestellt ist, dass diese den Verdampferkörper auf eine Temperatur aus einem Temperaturbereich zwischen etwa 100°C und etwa 140°C erhitzt. Ganz besonders bevorzugt beträgt die Temperatur im Verdampferbetrieb um etwa 120°C oder weniger, um die Bildung von geschlossenen, nicht schlagartig verdampfenden Tropfen auf der Verdampferoberfläche optimal zu verhindern. Besonders zweckmäßig ist es, wenn der maximale Durchmesser der vorzugsweise zumindest abschnittsweise zylindrisch konturierten Sacklöcher ein Maß von 50mm nicht überschreitet. Noch weiter bevorzugt ist es, wenn der maximale Durchmesser 40mm oder weniger beträgt. Ganz besonders bevorzugt ist der Durchmesser aus einem Wertebereich zwischen etwa 30 mm und 35 mm gewählt. Idealerweise ist auch die maximale Tiefenerstreckung der (unten geschlossenen) Sacklöcher begrenzt und beträgt weniger als 50mm, ganz besonders bevorzugt weniger als 40mm, noch weiter bevorzugt weniger als 30mm. Bevorzugt beträgt die Tiefenerstreckung bis zum Sacklochgrund etwa 25mm.

In Weiterbildung der Erfindung ist mit Vorteil vorgesehen, dass die Querschnittsfläche der Sacklöcher zumindest abschnittsweise, vorzugsweise über den größten Teil von deren Axialerstreckung, vorzugsweise über deren gesamte Axialerstreckung konstant ist.

Besonders bevorzugt ist eine Ausführungsvariante, bei der die Sacklöcher nicht kreisrund konturiert sind, sondern rechteckig, insbesondere, zumindest näherungsweise, quadratisch, wobei es noch weiter bevorzugt ist, wenn die Sacklöcher hierbei keine scharfen, sondern einen Radius aufweisende, d.h. gerundete Kanten haben. Diese Ausführungsvariante scheint auf den ersten Blick seltsam, da rechteckig konturierte Sacklöcher im Vergleich zu kreisrund konturierten Sacklöchern, vergleichsweise aufwändig, insbesondere durch Fräsen, zu fertigen sind. Durch die rechteckige Kontur (zwei rechtwinklig angeordnete Parallelseitenpaare), vorzugsweise mit gerundeten Ecken, können jedoch überraschend optimierte Verdampfungseigenschaften realisiert werden.

Besonders zweckmäßig ist es, wenn das Längen- zu Breitenverhältnis der Sacklöcher etwa 1 bis etwa 1,3, besonders bevorzugt etwa 1,1 beträgt. In einer ganz besonders bevorzugten Ausführungsvariante beträgt die Länge jedes Sackloches (gemessen in Richtung der Längserstreckung des Verdampfers) etwa 27,75mm und (die sich senkrecht dazu erstreckende Breite) beträgt etwa 25mm. Besonders bevorzugt beträgt die Tiefe der rechteckig konturierten Sacklöcher etwa 24mm. Anders ausgedrückt beträgt das Verhältnis zwischen Länge, Breite und Tiefe näherungsweise 1:1:1. Als besonders vorteilhaft hat es sich herausgestellt, wenn die rechteckig konturierten Sacklöcher in einer Reihe angeordnet sind. Ganz besonders bevorzugt sind sechs identische Sacklöcher in Reihe, d.h. in Richtung der Längserstreckung des Verdampfers hintereinander angeordnet, wobei jeweils zwei benachbarte Sacklöcher bevorzugt weniger als 10mm, ganz besonders bevorzugt etwa 4mm, voneinander beabstandet angeordnet sind.

Bevorzugt ist die Anzahl der vorgesehenen und/oder mit zu verdampfender Dekontaminationsflüssigkeit zu versorgender Sacklöcher an die zu erzielende Wirkstoffkonzentration in der Trägerluft und/oder in der Atmosphäre des zu dekontaminierenden Raumes angepasst bzw. derart abgestimmt. Gemäß der Erfindung sind den Sacklöchern jeweils mindestens ein, vorzugsweise ausschließlich jeweils ein Zuführkanal zum, insbesondere tropfenweisen Zuführen von zu verdampfenden Dekontaminationsmitteln zugeordnet - das bedeutet, dass die Zuführkanäle derart angeordnet sind, dass das zu verdampfende Dekontaminationsmittel unmittelbar in die Sacklöcher zuführbar, vorzugsweise in diese eintropfbar ist.

Im Hinblick auf die geometrische Ausgestaltung der Sacklöcher gibt es unterschiedliche Möglichkeiten. Ganz besonders bevorzugt weisen diese eine zylindrische Mantelfläche, bevorzugt gepaart mit einer kreisflächigen Bodenfläche auf. Alternativ können die Sacklöcher eine zylindrische Mantelfläche, gepaart mit einem konusförmigen Bodenabschnitt aufweisen.

Wie zuvor bereits ausgeführt, sind die Sacklöcher gemäß einer alternativen Ausführungsvariante rechteckig, insbesondere zumindest näherungsweise quadratisch konturiert, wobei es noch weiter bevorzugt ist, wenn die in Hochrichtung verlaufenden Kanten (in Umfangsrichtung betrachtet) gerundet ausgeführt sind, beispielsweise und bevorzugt mit einem Krümmungsradius von etwa R = 5mm.

Gemäß einer alternativen Ausführungsvariante nimmt der Durchmesser der bevorzugt kreisrund konturierten Querschnittsfläche der Sacklöcher mit zunehmendem Abstand von deren oberer Einlassöffnung ab, alternativ gestuft oder kontinuierlich, beispielsweise konisch.

Besonders bevorzugt ist es, wenn sämtliche Sacklöcher identisch ausgeformt sind.

In Weiterbildung der Erfindung ist mit Vorteil vorgesehen, dass die Sacklöcher nicht unmittelbar aneinander angrenzen, sondern voneinander beabstandet sind, insbesondere um hierdurch eine gleichmäßige Wärmezuführung zu gewährleisten. Bevorzugt sind die Sacklöcher nicht unmittelbar, etwa über Querbohrungen hydraulisch miteinander verbunden.

Auch im Hinblick auf die konkrete Ausgestaltung des Verdampferkörpers gibt es unterschiedliche Möglichkeiten. In der Regel sind bekannte Verdampferkörpers zylindrisch konturiert. Bevorzugt ist eine Ausführungsvariante, bei der der, vorzugsweise zumindest abschnittsweise quaderförmig konturierte Verdampferkörper der hier vorgeschlagenen Vorrichtung zur Erzeugung von Dekontaminationsmitteldampf eine größere Längen- als Breitenerstreckung und bevorzugt auch eine größere Längen- als Tiefenerstreckung aufweist. Ganz besonders zweckmäßig ist es, wenn der Verdampferkörper, zumindest abschnittsweise, zumindest näherungsweise, quaderförmig konturiert ist.

Auch im Hinblick auf die Anordnung der Sacklöcher gibt es unterschiedliche Möglichkeiten. Bevorzugt ist eine Ausführungsvariante, bei der mindestens eine, vorzugsweise ausschließlich eine sich in Richtung der Längserstreckung des Verdampferkörpers erstreckende Reihe von Sacklöchern vorgesehen ist. Es ist auch eine Ausführungsform mit mehreren, insbesondere parallelen Reihen realisierbar.

Die Heizeinrichtung ist bevorzugt derart ausgebildet und angeordnet, dass diese sich entlang der Längsseiten, d.h. entlang der Längserstreckung des Verdampferkörpers erstreckt. Bevorzugt erstreckt sie sich zusätzlich auch entlang der Bodenfläche. Ganz besonders zweckmäßig ist es im Falle des Vorsehens von zwei, insbesondere parallelen Sacklochreihen, wenn sich die Heizeinrichtung in einen Bereich zwischen die Reihen hineinerstreckt, um somit eine gleichmäßige Wärmezufuhr zu den Sacklöchern zu gewährleisten. Bevorzugt handelt es sich bei der Heizeinrichtung um eine elektrische Widerstandsheizung. Zweckmäßig ist im Verdampferkörper ein Temperatursensor, beispielsweise ein PT 100, aufgenommen. Der Temperatursensor, insbesondere der PT 100, dient bevorzugt zur Überwachung bzw. zur Aufzeichnung der Temperatur bzw. des Temperaturverlaufs. Ganz besonders bevorzugt ist zusätzlich oder alternativ zum Temperatursensor ein der Heizeinrichtung zugeordnetes Thermoelement vorgesehen. Idealerweise handelt es sich bei der Heizeinrichtung um mindestens einen Heizstab, der bevorzugt in einer sich in Richtung der Längserstreckung des Verdampfers verlaufenden Öffnung, insbesondere Bohrung, aufgenommen ist. Bevorzugt ist in dieser Öffnung auch das Thermoelement angeordnet, bevorzugt integral mit dem Heizstab. Noch weiter bevorzugt ist es, wenn mehrere, insbesondere parallel angeordnete Heizstäbe vorgesehen sind. Gute Ergebnisse wurden mit insgesamt drei Heizstäben erzielt, wobei die drei Heizstäbe bevorzugt mittels eines gemeinsamen Thermoelementes geregelt werden, welches sich noch weiter bevorzugt zusammen mit dem mittleren Heizstab in einer mittleren Bohrung bzw. Öffnung befindet. Besonders bevorzugt ist der Einsatz von insgesamt nur zwei, symmetrisch bezogen auf eine senkrechte Spiegelebene, angeordneten Heizstäben.

Wie eingangs erwähnt sind die, vorzugsweise starren Zuführkanäle den Sacklöchern zugeordnet, um möglichst jedem Sackloch Dekontaminationsflüssigkeit zuführen zu können. Um zu verhindern, dass die durch die Zuführkanäle zugeführte Dekontaminationsflüssigkeit von Trägerluft mitgerissen werden kann, bevor die Flüssigkeit verdampft ist, ist in Weiterbildung der Erfindung mit Vorteil vorgesehen, dass die Zuführkanäle die Einlassöffnungen der Sacklöcher durchsetzen, also in die Sachlöcher hineinragen. Ganz besonders bevorzugt ist es dabei, wenn die, bevorzugt getrocknete und erwärmte, Trägerluft die Sacklöcher überströmt und auf diese Weise den aus den Sacklöchern aufsteigenden Dekontaminationsmitteldampf mitführt.

In der Praxis kann es vorkommen, dass die Zuführkanäle verkrusten oder einem Verschleiß unterliegen. Um größere Reparaturarbeiten zu vermeiden, ist daher in Weiterbildung der Erfindung mit Vorteil vorgesehen, dass die Zuführkanäle, durch die die zu verdampfende Dekontaminationsflüssigkeit, vorzugsweise tropfenförmig zu den Sacklöchern geleitet wird austauschbar, d.h. auswechselbar ausgebildet und angeordnet sind. Überraschenderweise kann hierzu zur Ausbildung der vorzugsweise starren, insbesondere metallische, Zuführkanäle auf als Massenware erhältlich, insbesondere aus Edelstahl ausgebildete Injektionsnadel zurückgegriffen werden, welche bevorzugt an ihrem oberen, von den Sacklöchern abgewandten Enden mit einem Luer-Lock-Anschluss versehen sind, über den die Austauschbarkeit bzw. Auswechselbarkeit gewährleistet werden kann. Besonders bevorzugt ist der Innendurchmesser der Zuführkanäle aus einem Wertebereich zwischen etwa 0,5mm und etwa 2mm gewählt.

Bevorzugt sind die, insbesondere von Injektionsnadeln gebildeten Zuführkanäle derart angeordnet, dass sie zu verdampfende Dekontaminationsflüssigkeit aus einer Verteilerkammer den Sacklöchern zuführen, also die hydraulische Verbindung zwischen der Verteilerkammer und dem jeweiligen Sackloch darstellen. Hierzu ragen die Zuführkanäle bevorzugt in einen oberhalb der Sacklöcher und damit oberhalb des Verdampferkörpers angeordneten Strömungskanal für Trägerluft hinein bzw. sind in diesem angeordnet oder durchsetzen diesen bevorzugt bis in die Sacklöcher hinein, um wie zuvor bereits erläutert, ein Mitreißen von noch nicht verdampfter Dekontaminationsflüssigkeit mit der Trägerluft zu verhindern.

Besonders zweckmäßig ist eine Ausführungsvariante, bei der die zu verdampfende Dekontaminationsflüssigkeit zu den Zuführkanälen über jeweils einen Versorgungskanal strömen kann. Idealerweise handelt es sich bei diesen Versorgungskanälen nicht um Schlauchleitungen, sondern um in ein metallisches Verdampferbauteil eingebrachte, insbesondere eingefräste, Kanäle. Idealerweise sind die Versorgungskanäle in einer Deckelplatte eingebracht und verbinden eine, insbesondere zentrale, Versorgungsleitung mit den Zuführkanälen. Um zu gewährleisten, dass die Zuführkanäle jeweils mit der gleichen Menge an zu verdampfendem Dekontaminationsmittel versorgt werden, ist in Weiterbildung der Erfindung mit Vorteil vorgesehen, dass die Kanäle - bei gleichem Durchflussquerschnitt - gleich lang ausgebildet sind, um somit der zu verdampfenden Flüssigkeit den gleichen Strömungswiderstand entgegenzusetzen. Idealerweise werden die vorgenannten Versorgungskanäle über eine getaktete, insbesondere außerhalb des eigentlichen Verdampfers angeordnete Dosierpumpe versorgt. Besonders zweckmäßig ist es, wenn die Versorgungskanäle aus einem, bevorzugt zentrischen Raum, ausmünden, der noch weiter bevorzugt als Vertiefung in der die Versorgungskanäle aufweisenden Deckelplatte eingebracht ist. Die Versorgungskanäle können beispielsweise über eine gemeinsame Verschlussplatte verschlossen werden, die z.B. an der Deckelplatte durch Verschrauben festlegbar ist. Zusätzlich oder alternativ können die Versorgungskanäle auf ihrer Oberseite mit Metallblechen, insbesondere Edelstahlblechen, verschlossen sein, wobei diese Bleche bevorzugt mit der die Versorgungskanäle aufweisenden Deckelplatte verschweißt sind.

Besonders zweckmäßig ist es, wenn sich die Durchflussmenge der zu verdampfenden Dekontaminationsflüssigkeit für die einzelnen Zuführkanäle einstellen lässt. Anders ausgedrückt ist es bevorzugt, wenn zumindest einigen der Zuführkanäle eine Einstelleinrichtung, insbesondere eine Einstellschranke, zugeordnet ist, mit der die Durchflussmenge, die den Zuführkanal durchströmen soll einstellbar ist. Bevorzugt befinden sich diese Einstelleinrichtungen innerhalb oder im Bereich der vorerwähnten Verteilerkammer, in welche zu verdampfende Dekontaminationsflüssigkeit bevorzugt aus einem Vorrat, insbesondere einer austauschbaren Vorratsflasche gepumpt werden kann.

Die Erfindung betrifft auch eine Anordnung, insbesondere eine pharmazeutische Anordnung, umfassend einen mit Dekontaminationsmitteldampf zu dekontaminierenden Raum, insbesondere einen Isolator und/oder eine Schleuse sowie eine wie zuvor beschrieben ausgebildete Vorrichtung zur Erzeugung von Dekontaminationsmitteldampf, insbesondere von Wasserstoffperoxyddampf.

Im Stand der Technik werden Schnellverdampfer üblicherweise derart angeordnet, dass diese mit Luft aus dem zu dekontaminierenden Raum als Trägerluft versorgt werden und die mit Wasserstoffperoxyd angereicherte Trägerluft unmittelbar wieder in den zu dekontaminierenden Raum abgeben. Dies führt jedoch in der Regel zu einer Kondensation von Wasserstoffperoxyd in dem zu dekontaminierenden Raum. Eine solche Kondensation soll in Weiterbildung der Erfindung möglichst verhindert werden, was zweckmäßigerweise dadurch unterstützt bzw. erreicht werden kann, dass der Schnellverdampfer nicht, oder nicht nur, mit Trägerluft aus dem zu dekontaminierenden Raum versorgt wird, sondern mit Frischluft. Es wird unter Frischluft nicht nur, bevorzugt getrocknete, Umgebungsluft (insbesondere <10% relative Luftfeuchte), sondern auch Druckluft aus entsprechenden Vorratsbehältern, insbesondere Druckflaschen verstanden. Wesentlich ist, dass es sich nicht um aus dem zu dekontaminierenden Raum zugeführte Luft handelt. Hierzu ist es notwendig, dass auch kontinuierlich Luft aus dem dem zu dekontaminierenden Raum, beispielsweise an die Umgebung abgegeben wird, um unzulässige Überdrücke zu vermeiden. Der Vorteil einer derartigen Anordnung besteht in der besseren Regelbarkeit der Wasserstoffperoxydkonzentration in der Raumluft des zu dekontaminierenden Raumes und einer besseren Druckregelbarkeit während der Dekontamination. Der Druck im Raum sollte während der Dekontamination über Umgebungsdruck liegen, vorzugsweise um etwa 30Pa darüber. Im Hinblick auf die konkrete Anordnung des Verdampfers gibt es dabei zwei unterschiedliche Möglichkeiten. So kann der Verdampfer beispielsweise an die einzige Frischluftzuführleitung (Trägerluftzuführleitung) angeschlossen werden, so dass sämtliche in den zu dekontaminierenden Raum zuzuführende Luft den Verdampfer unmittelbar durchströmt. Bevorzugt ist es den Verdampfer auf diese Weise anzuordnen, wenn die Frischluftzufuhr im Betrieb des zu dekontaminierenden Raumes, insbesondere eines Isolators, etwa 80m³ pro Stunde nicht übersteigt. Bei einer alternativen Anordnung ist der, vorzugsweise einzigen in Strömungsrichtung vor dem Verdampfer angeordneten, Frischluftleitung (Zuführleitung) eine Bypassleitung zugeordnet, an die der Verdampfer anzuschließen ist - dies führt dazu, dass ein Teil der zugeführten Frischluft nicht unmittelbar den Verdampfer durchströmt sondern, vorzugsweise vor Erreichen des zu dekontaminierenden Raumes - mit der abgezweigten, durch den Verdampfer strömenden, angereicherten Trägerluft vermischt wird. Eine derartige Anordnung ist insbesondere dann sinnvoll, wenn der zu dekontaminierende Raum im Betrieb, d.h. nach der Dekontamination eine Fristluftzufuhr von mehr als 80m³ pro Stunde.

Zur Einstellung bzw. Regelung der Dekontaminationsmitteldampfkonzentration in der Trägerluft ist es bevorzugt, wenn in eine dem Verdampfer nachgeordnete Zwischen- bzw. Anschlussleitung eine Regulationsleitung mündet, über die eine einstellbare Luftmenge, insbesondere Frischluftmenge, zur Verdünnung der Dampfkonzentration zur Trägerluft zugemischt werden kann.

Weitere Vorteile, Merkmale und Einzelheiten der Erfindung ergeben sich aus der nachfolgenden Beschreibung bevorzugter Ausführungsbeispiele sowie anhand der Zeichnungen. Diese zeigen in:
- Fig. 1:: eine perspektivische Ansicht einer Vorrichtung zur Erzeugung von Dekontaminationsmitteldampf (Schnellverdampfer, Blitzverdampfer),
- Fig. 2 bis Fig. 5:: unterschiedliche Schnittansichten der Vorrichtung gemäß Fig. 1,
- Fig. 6:: die Darstellung eines alternativen Verdampfers,
- Fig. 7 bis Fig. 10:: unterschiedliche Schnittansichten der Vorrichtung gemäß Fig. 6,
- Fig. 11:: in einer stark schematisierten Ansicht eine pharmazeutische Anordnung, umfassend einen Isolator und einen Schnellverdampfer, wobei die einzige Frischluftzuleitung direkt an den Verdampfer angeschlossen ist,
- Fig. 12:: eine alternative pharmazeutische Anordnung, bei welcher der Verdampfer in einer Bypass-Leitung in einer Frischluftleitung angeordnet ist.
- Fig. 13:: in einer Explosionsdarstellung eine weitere alternative Ausführungsform eines Verdampfers,
- Fig. 14a bis Fig. 14e:: den Verdampferkörper des Verdampfers gemäß Fig. 13 in verschiedenen, teilweise geschnittenen Ansichten,
- Fig.15a bis Fig. 15e:: unterschiedliche, teilweise geschnittene Ansichten einer Verschlussplatte zur Befestigung der in Fig. 16a bis Fig. 16e gezeigten Deckelplatte, und
- Fig. 16a bis Fig. 16e:: eine Deckelplatte des Verdampfers gemäß Fig. 13 mit in diese eingebrachten, gleich langen Versorgungskanälen.

In den Figuren sind gleiche Elemente und Elemente mit der gleichen Funktion mit den gleichen Bezugszeichen gekennzeichnet.

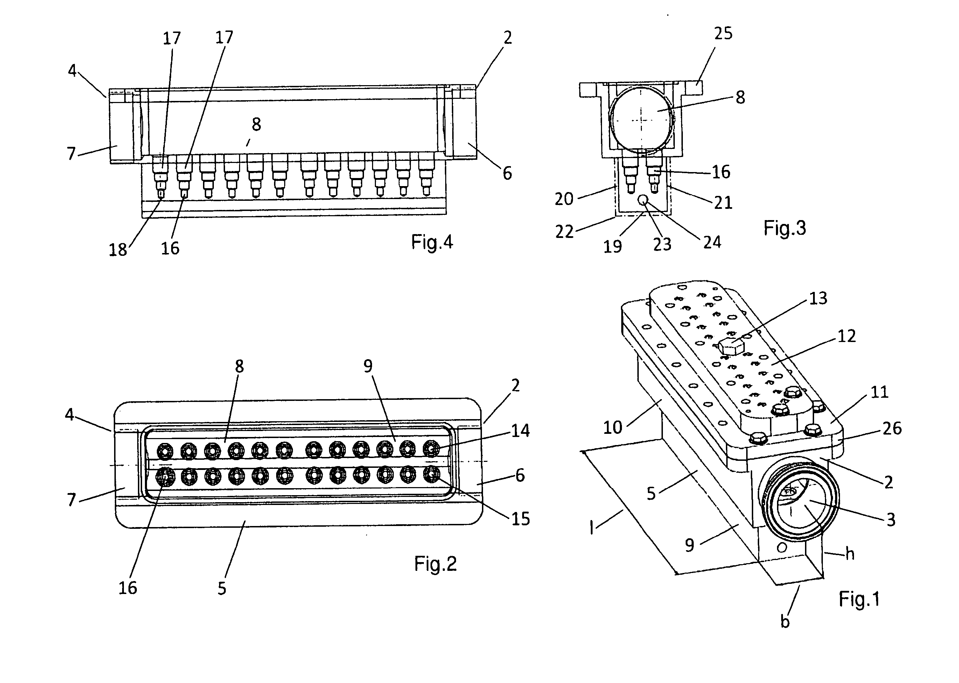

Fig. 1 zeigt ein erstes Ausführungsbeispiel einer Vorrichtung 1 (Schnellverdampfer, Blitzverdampfer) zur Erzeugung von Dekontaminationsmitteldampf, hier von Wasserstoffperoxyddampf zur Dekontamination einer pharmazeutischen, in einem Isolator angeordneten Verpackungsvorrichtung für Medikamente (nicht gezeigt).

Die Vorrichtung 1 umfasst an einem ersten Ende 2 einen Anschluss 3 für Trägerluft zum Abtransportieren des erzeugten Dekontaminationsmitteldampfes zu einem nicht dargestellten zu dekontaminierenden Raum. An dem von dem ersten Ende 2 abgewandten zweiten Ende 4 (vgl. Fig. 2) befindet sich ein in Fig. 1 nicht dargestellter Anschluss zum Anschließen einer zu dem zu dekontaminierenden Raum führenden Anschlussleitung. Die Anschlüsse sind in einem unteren Vorrichtungsteil 5 bzw. in dort eingebrachte Innengewindelöcher 6, 7 einschraubbar.

Axial zwischen den Anschlüssen ist ein freier Querschnitt, d.h. ein Strömungskanal 8 realisiert, durch den die Trägerluft, in diesem Fall Frischluft, beispielsweise Druckluft oder Umgebungsluft, strömen und dabei den Dekontaminationsmitteldampf mit sich nehmen kann.

Der untere Vorrichtungsabschnitt 5 umfasst einen metallischen Verdampferkörper 9 aus einer Aluminiumlegierung. Wie sich aus Fig. 1 ergibt, ist dessen Breitenerstreckung b wesentlich geringer als dessen Längenerstreckung l, wobei die Breitenerstreckung b in etwa der Höhenerstreckung h entspricht. Wie sich aus den Figuren weiter entnehmen lässt, ist der Verdampferkörper 9 quaderförmig ausgebildet und in dem gezeigten Ausführungsbeispiel einteilig ausgeführt mit der darüber befindlichen metallischen Ummantelung 10 des Strömungskanals 8. Mit dem unteren Vorrichtungsabschnitt 5 ist ein oberer Vorrichtungsabschnitt 11 deckelartig verschraubt, wobei der obere Vorrichtungsabschnitt 11 eine Verteilerkammer 12 umfasst, in welche über einem in dem gezeigten Ausführungsbeispiel mittels einer Transportschraube 13 verschlossenen Anschluss zu verdampfende Dekontaminationsflüssigkeit, insbesondere eine wässrige, vorzugsweise 35%-ige Wasserstoffperoxydlösung zugeführt werden kann.

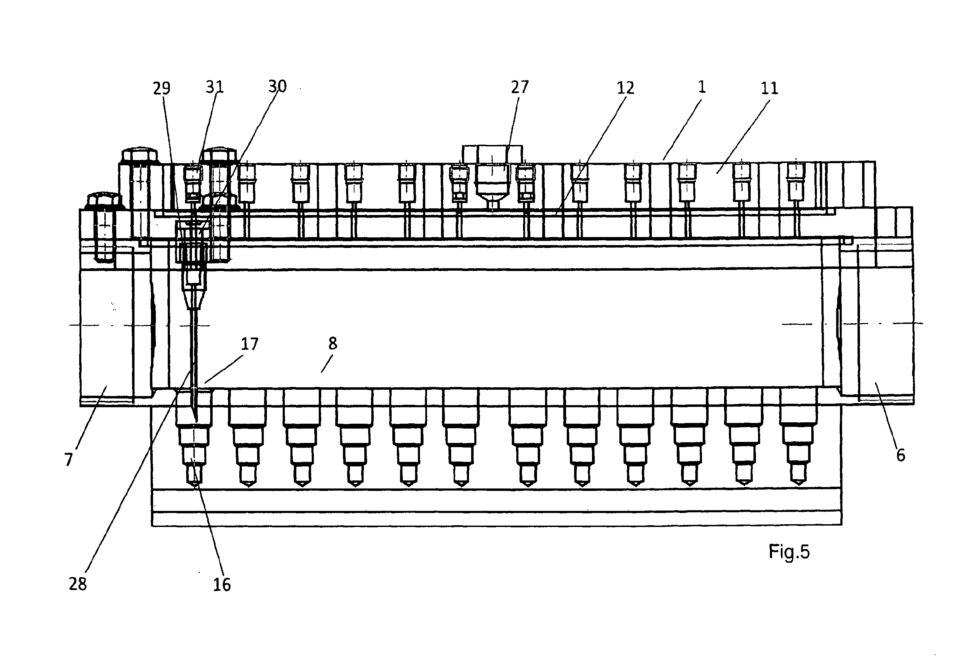

In den Verdampferkörper 9 sind in dem gezeigten Ausführungsbeispiel zwei Reihen 14, 15 von voneinander separaten und voneinander beabstandeten Sacklöchern 16, d.h. unten geschlossenen Löchern, eingebracht. Die Sacklöcher 16 bzw. Vertiefungen sind voneinander separat, d.h. nicht unmittelbar hydraulisch miteinander verbunden und erstrecken sich senkrecht zur Trägerluftströmungsrichtung und damit senkrecht zur Lösungserstreckung des Verdampferkörpers 9. Wie sich insbesondere aus Fig. 4 ergibt, verringert sich die Querschnittsfläche der Sacklöcher 16 ausgehend von einer Einlassöffnung 17 bis hin zu einem Sacklochgrund 18. Bei dem gezeigten Ausführungsbeispiel sind die Sacklöcher 16 hierzu als mehrstufige Stufenbohrung realisiert, wobei alternativ auch eine konische Kontur realisierbar ist. Wie später noch anhand von Fig. 5 erläutert werden wird, ist der Strömungskanal 8 senkrecht zu seiner Längserstreckung durchsetzt von nadelartigen Zuführkanälen, durch welche den einzelnen Sacklöchern 16 tropfenweise zu verdampfender Dekontaminationsflüssigkeit aus der Verteilerkammer 12 zugeführt wird. Dabei durchsetzen die austauschbar angeordneten Zuführkanäle die Einfassöffnungen 17, so dass die durch den Strömungskanal 8 strömende Trägerluft diese nicht mitreißen kann.

Aus Fig. 3 ergibt sich, dass der Verdampferkörper 9 an seiner Bodenseite 19 sowie an beiden Längsseiten 20, 21 umgeben ist von einer, hier elektrischen, an den Verdampferkörper 9 anliegenden Heizeinrichtung 22, die derart regelbar ist, dass der Verdampferkörper eine Temperatur von dem gezeigten Ausführungsbeispiel etwa 110°C erreicht. Die Ist-Temperatur kann gemessen werden mit einem Temperaturmessfühler 23, der in einer sich in Richtung der Längserstreckung des Verdampferkörpers 9 erstreckenden Bohrung 24 aufnehmbar ist.

Aus den Fig. 1, 2 und 3 ergibt sich, dass der untere Verdampferabschnitt 5 an seiner Oberseite einen umlaufenden Befestigungsflansch 25 anbietet, der auf dem oberen Vorrichtungsabschnitt 11 mit einem entsprechenden Gegenflansch 26 aufliegt und verschraubt ist.

Fig. 5 zeigt die montierte Vorrichtung 1 in einer Längsschnittansicht. Zu erkennen ist der obere zentrische Anschluss 27 für eine Zuführleitung zum Zuführen von zu verdampfender Dekontaminationsflüssigkeit in die flache Verteilerkammer 12. Zur Ausbildung der Verteilerkammer 12 ist der obere Vorrichtungsabschnitt 11 bzw. dessen Gehäusewandung zweiteilig ausgeführt.

Wie sich weiter aus Fig. 5 ergibt, ist jeder Sacklochbohrung 16 ein einziger austauschbar ausgebildeter und angeordneter Zuführkanal 28 zugeordnet, der in dem gezeigten Ausführungsbeispiel jeweils von einer Injektionsnadel aus Edelstahl gebildet ist. Diese weist an ihrem oberen Abschnitt einen Luer-Lock-Anschluss 29 auf, mit welchem sie an einer entsprechenden Aufnahme 30 am oberen Vorrichtungsabschnitt 11 unterhalb der Verteilerkammer 12 festlegbar ist. Aus Übersichtlichkeitsgründen ist nur ein einziger Zuführkanal 28 mit zugehöriger Aufnahme 30 dargestellt - in der Realität ist jedem Sackloch 16 eine solche Aufnahme 30 mit zugehörigem Zuführkanal 28 zugeordnet. Zu erkennen ist, dass der Zuführkanal 28 den Strömungskanal 8 senkrecht zu seiner Längserstreckung durchsetzt und unterhalb der Einlassöffnung 17 in das Sackloch 16 mündet. Jedem Zuführkanal 28 ist eine Einstellschraube 31 zugeordnet, mit welcher die Flüssigkeitsmenge eingestellt werden kann, die durch den Zuführkanal 28 hin zu dem jeweiligen Sackloch 16 strömt.

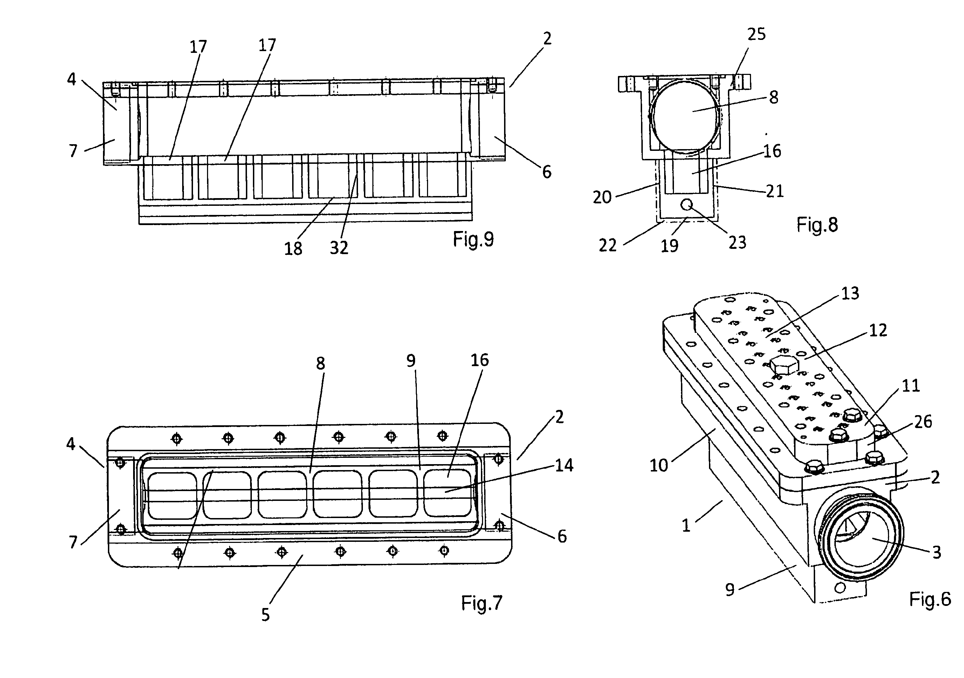

Die in den Fig. 6 bis 10 dargestellte Vorrichtung 1 entspricht im Wesentlichen der in den Fig. 1 bis 5 gezeigten und zuvor beschriebenen Vorrichtung. Zur Vermeidung von Wiederholungen wird im Folgenden im Wesentlichen nur auf die Unterschiede eingegangen - hinsichtlich der Gemeinsamkeiten wird auf die Fig. 1 mit zugehöriger Figurenbeschreibung verwiesen.

Im Unterschied zu dem zuvor beschriebenen Ausführungsbeispiel umfasst der Verdampferkörper 9 der Vorrichtung 1 gemäß den Fig. 6 bis 10 lediglich eine einzige Reihe 14 von Sacklöchern 16. Diese sind nicht als Stufenbohrungen oder konisch ausgeführt, sondern umfassen eine zylindrische Mantelfläche 32, wobei der Sacklochgrund 18 bevorzugt wie dargestellt kreisscheibenförmig ausgeformt ist - zur vereinfachten Herstellung ist es jedoch auch denkbar einen konusförmigen Sacklochgrund zu realisieren. In dem gezeigten Ausführungsbeispiel sind insgesamt sechs Sacklöcher vorgesehen. Ganz besonders bevorzugt ist die Realisierung von nur fünf Sacklöchern, wobei es ganz besonders bevorzugt ist, wenn jedem Sackloch 16 ein einziger, bevorzugt von einer Injektionsnadel gebildeter, Zuführkanal 28 zugeordnet ist.

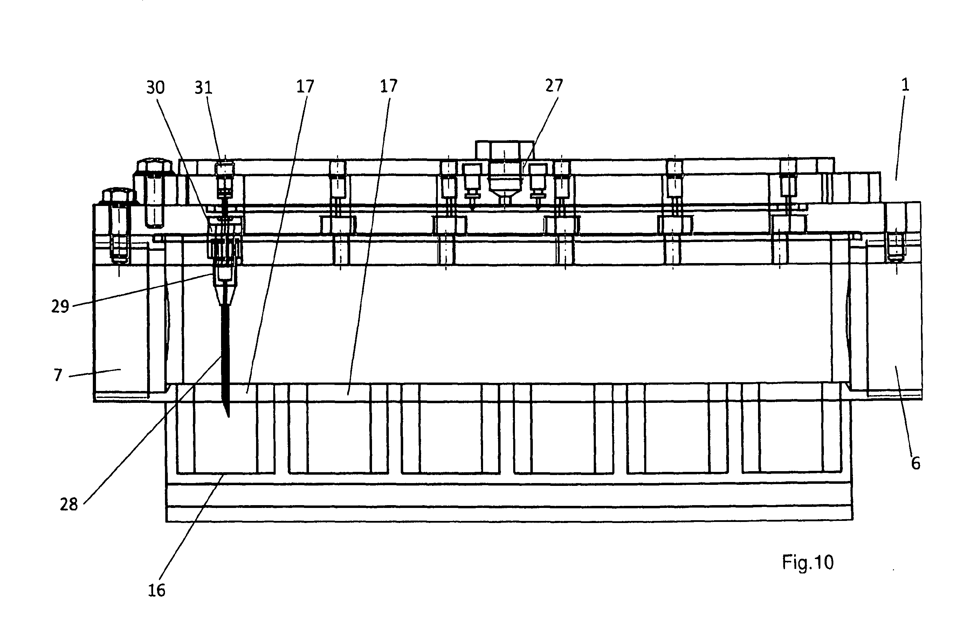

Fig. 10 zeigt aus Übersichtlichkeitsgründen wieder nur einen einzigen Zuführkanal 28, wobei jedem Sackloch 16 ein eigener, hier mit Luer-Lock-Anschluss 29 versehener Zuführkanal 28 zugeordnet ist, der in einer zugehörigen Aufnahme 30 austauschbar gehalten ist.

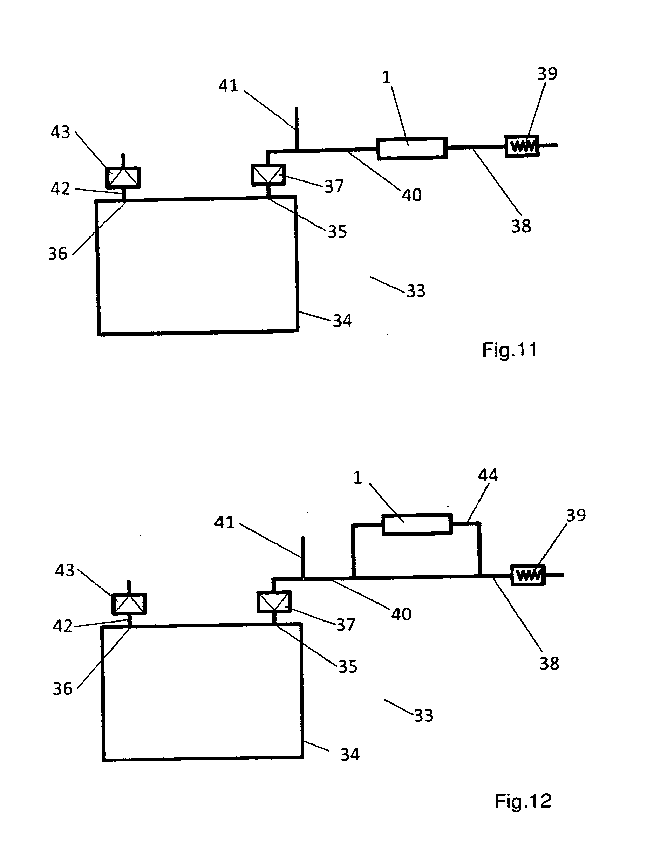

Fig. 11 zeigt eine Anordnung 33, hier eine pharmatechnischen Anordnung, umfassend einen lediglich stark schematisiert dargestellten, als Isolator ausgebildeten, zu dekontaminierenden Raum 34, umfassend einen Eingangsanschluss 35 und einen Ausgangsanschluss 36. Dem Eingangsanschluss 35 ist ein Filter 37, hier ein so genannter Hepa-Filter zugeordnet, zur Filterung der zuströmenden, mit Dekontaminationsmitteldampf angereicherten Trägerluft. Bei einer alternativen, nicht gezeigten, Ausführungsvariante kann direkt in den Raum, d.h. nicht über das Filter, Dekontaminationsmitteldampf zugeführt werden. An die einzige in Strömungsrichtung vor der Vorrichtung 1 angeordneten Frischluftleitung 38 ist eine Vorrichtung 1 (Schnellverdampfer) direkt angeschlossen, so dass die gesamte durch die Frischluftleitung 38 zuströmende Luft die Vorrichtung 1 durchströmt und dabei mit Dekontaminationsmitteldampf angereichert wird. Die Trägerluft (Frischluft) kann mittels einer Temperiereinrichtung 39 vorgewärmt werden. Die Frischluft kann beispielsweise einer Druckgasflasche entstammen oder aus der Umgebung angesaugt werden, wobei in letzterem Fall bevorzugt eine Lufttrocknungseinrichtung vorgesehen ist. In die Strömungsrichtung hinter der Vorrichtung 1 mündet in die Leitung 40 (Zwischenleitung) eine Regulierleitung 41 zur geregelten Zufuhr weiterer Frischluft zur Einstellung der Dekontaminationsmitteldampfkonzentration in der dem Raum 34 zuzuführenden Trägerluft. An den Ausgangsanschluss 36 ist eine Abführleitung 42 angeschlossen, in die ein Filter 43, hier ein Hepa-Filter, angeschlossen ist, über den die Abluft in die Umgebung oder zu einem Katalysator zur Zersetzung des Wasserstoffperoxyddampfes strömen kann.

Die Anordnung 33 gemäß Fig. 12 entspricht im Wesentlichen der Anordnung gemäß Fig. 11. Im Folgenden wird zur Vermeidung von Wiederholungen im Wesentlichen auf die Unterschiede eingegangen. Im Hinblick auf die Gemeinsamkeiten wird auf Fig. 11 mit zugehöriger Figurenbeschreibung verwiesen.

Zu erkennen ist, dass die Vorrichtung 1 (Schnellverdampfer) an eine Bypassleitung 44 zur Frischluftleitung 38 angeschlossen ist, so dass nicht sämtliche, zugeführt Frischluft die Vorrichtung 1 durchströmt. Wie bei dem Ausführungsbeispiel gemäß Fig. 11 basiert das Dekontaminationsprinzip auf einer so genannten Frischluftdekontamination - dies bedeutet, dass der Vorrichtung 1 ausschließlich nicht aus dem zu dekontaminierenden Raum 34 stammende, d.h. mit Wasserstoffperoxyddampf angereicherte, Luft zugeführt wird, sondern Frischluft, d.h. Druckluft oder, insbesondere getrocknete, Umgebungsluft.

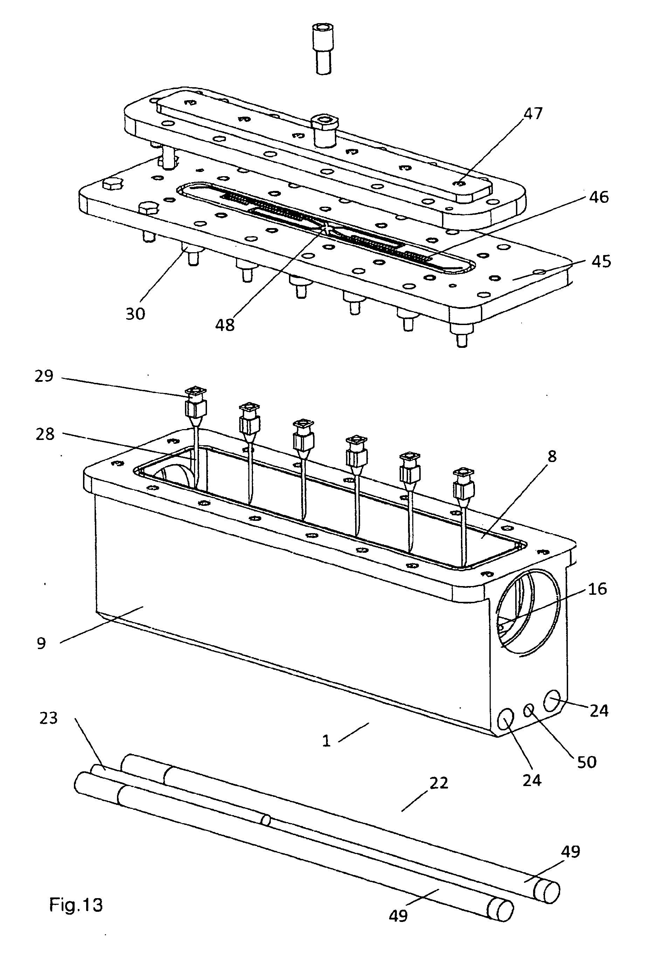

Fig. 13 zeigt ein alternatives Ausführungsbeispiel einer Vorrichtung 1 (Schnellverdampfer, Blitzverdampfer) zur Erzeugung von Dekontaminationsmitteldampf, hier von Wasserstoffperoxyddampf zur Dekontamination eines pharmazeutischen Isolators.

Die Vorrichtung 1 umfasst einen in einem oberen Bereich wannenartig ausgeformten und in einem unteren Bereich quaderförmigen Verdampferkörper 9, wobei der obere Bereich einen Strömungskanal 8 seitlich umschließt. An dem Verdampferkörper 9 ist eine Deckelplatte 45 durch Verschrauben festlegbar, die den Strömungskanal 8 im montierten Zustand oben verschließt. In die Deckelplatte 45 sind später noch zu erläuternde Versorgungskanäle 46, hier durch Fräsen, eingebracht. Die Versorgungskanäle 46 transportieren zu verdampfende Dekontaminationsflüssigkeit zu Aufnahmen 30 für auswechselbare Zuführkanäle 28. Letztere sind als Injektionsnadeln aus Edelstahl ausgebildet und weisen an ihrem oberen Ende einen Luer-Lock-Anschluss 29 zur Fixierung in jeweils einer Aufnahme 30 auf.

In dem gezeigten Ausführungsbeispiel verschließt die mit der Deckelplatte 16 verschraubbare Verschlussplatte 47 die nutartigen Versorgungskanäle 46 von oben. Zusätzlich oder alternativ können die Versorgungskanäle 46 mit einer oberen, vorzugsweise nachträglich eingeschweißten Wandung, insbesondere aus Edelstahl, verschlossen werden.

Die Versorgungskanäle 46 münden aus einer zentralen Vertiefung 48 (Raum) aus, in die zu verdampfende Dekontaminationsflüssigkeit von außen mittels einer getakteten Dosierpumpe (nicht gezeigt) über eine Versorgungsleitung zugeführt wird.

Wie sich aus Fig. 13 weiter ergibt, sind in dem Verdampferkörper 9 in einem Bereich unterhalb der später noch zu erläuternden Sacklöcher 16 drei parallel, sich in Richtung der Längserstreckung des Verdampferkörpers 9 erstreckende Bohrungen 24 eingebracht. In jede Bohrung 24 kann ein Heizstab 49 eingeführt werden. Die Heizstäbe 49 bilden die Heizeinrichtung 22. Bevorzugt wird bei dieser Ausführungsform auf eine Ummantelungsheizung (Mantelheizung) verzichtet. Bevorzugt ist dem mittleren Heizstab 49 ein nicht gezeigtes Thermoelement zugeordnet, mit dem die drei Heizstäbe 49 regelbar sind. Ferner ist in Fig. 13 ein PT 100 (Temperaturfühler 23) gezeigt, der in eine Öffnung 50 schräg oberhalb der mittleren Bohrung 24 einführbar ist.

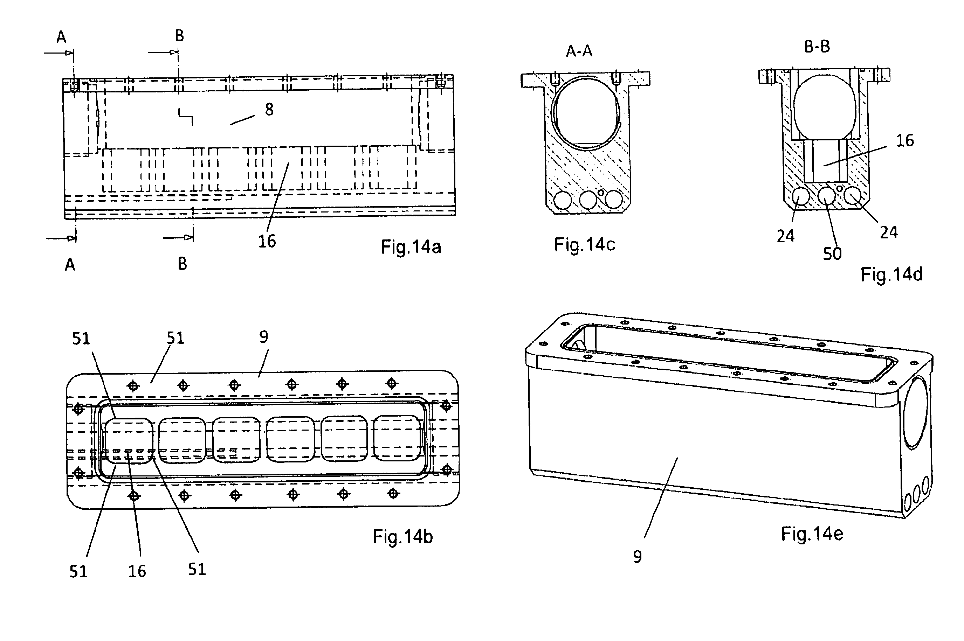

Fig. 14a bis 14c zeigen unterschiedliche Darstellungen des Verdampferkörpers 9 gemäß dem Ausführungsbeispiel nach Fig. 13. Zu erkennen sind die in einer Draufsicht rechteckig, hier näherungsweise quadratisch, konturierten Sacklöcher 16, wobei insgesamt sechs Sacklöcher in Reihe angeordnet sind. Das Verhältnis zwischen Sacklochlänge, Sacklochbreite und Sacklochtiefe beträgt in etwa 1:1:1. Die Sachlochlänge beträgt 27,75mm, die Sacklochbreite 25mm und die Sachlochtiefe 24mm. Wie sich aus Fig. 14b ergibt, sind die Ecken (Kanten 51) der Sacklöcher 16 gerundet ausgeführt. Jedem Sachloch 16 ist ein in Fig. 13 gezeigter, auswechselbarer Zuführkanal 28 zugeordnet. Auch bei dem Ausführungsbeispiel gemäß den Fig. 13 bis 16d ragen die Zuführkanäle 28 in das jeweilige Sackloch 16 hinein, durchsetzen also den Strömungskanal 8, um ein Mitreißen von Flüssigkeitstropfen zu vermeiden.

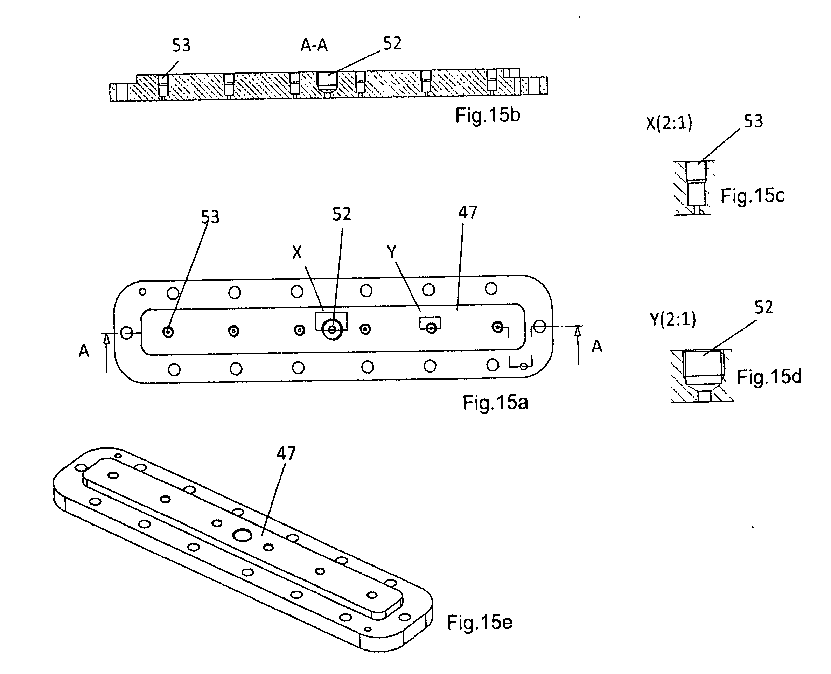

Fig. 15a bis 15e zeigen den Verschlussdeckel 47. Dieser weist eine zentrische Zuführung 52 auf, durch die zu verdampfende Dekontaminationsflüssigkeit in den Verdampfer gelangt. Wie sich aus Fig. 15d ergibt, ist die Zuführung 52 als Innengewindeloch ausgeführt, um einen entsprechenden Zuführschlauch (Versorgungsleitung) oder einen Adapter für einen derartigen Schlauch festlegen zu können.

Ferner sind sechs in Reihe angeordnete Innengewindelöcher 53 vorgesehen, wobei jedem Zuführkanal 28 (vgl. Fig. 13) eine derartige Innengewindeloch 53 zugeordnet ist. Diese nehmen jeweils eine Stellschraube (nicht gezeigt) zur Einstellung der zuzuführenden Dekontaminationsflüssigkeitsmenge auf. Die Einstellschraube ist aus Übersichtlichkeitsgründen nicht gezeigt.

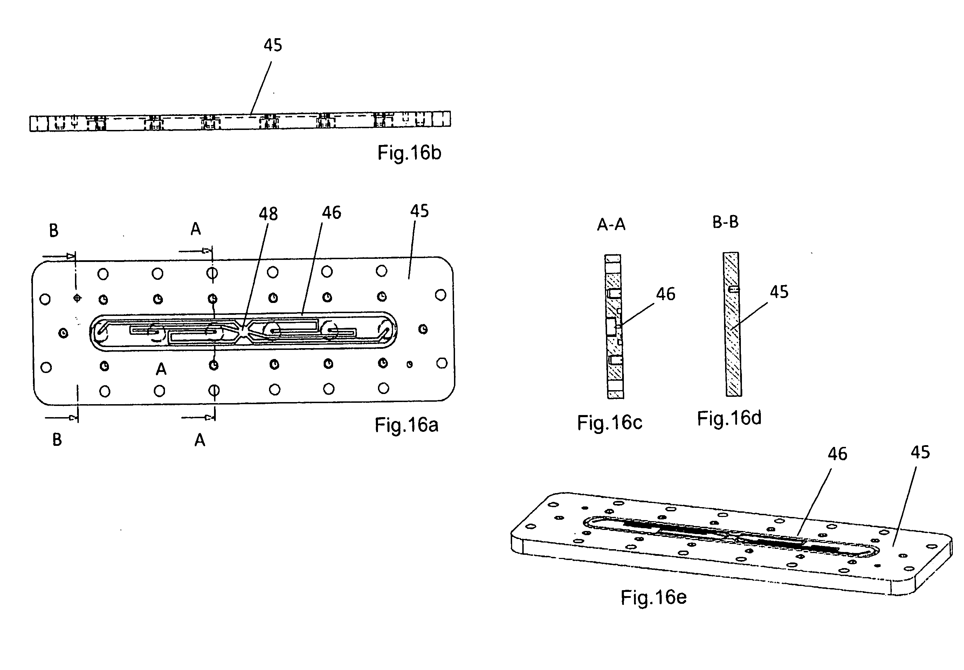

Fig. 16a bis 16e zeigen die in Fig. 13 gezeigte Deckelplatte 45, auf die die in den Fig. 15a bis 15e gezeigte Verschlussplatte 47 aufschraubbar ist, um die in die Deckelplatte 45 eingebrachten Versorgungskanäle 46 zu verschließen. Sämtliche Versorgungskanäle 46 sind gleich lang und weisen den gleichen Kanalquerschnitt auf. Die Versorgungskanäle 46 münden aus einer Vertiefung 48 aus, die im montierten Zustand unterhalb der Zuführung 52 angeordnet ist. Aus der Vertiefung 48 kann über die Versorgungskanäle 46 zu verdampfende Dekontaminationsflüssigkeit zu den Zuführkanälen 28 (vgl. Fig. 13) strömen. Um eine gleiche Länge der Versorgungskanäle 46 zu realisieren, sind diese unterschiedlich, teilweise mit Richtungswechsel ausgeformt. Die Versorgungskanäle 46 haben in dem gezeigten Ausführungsbeispiel einen rechteckigen Kanalquerschnitt (vgl. Fig. 16c). Im montierten Zustand verschließt die Deckelplatte 45 den Strömungskanal 8 für Frischluft, beispielsweise Druckluft oder getrocknete Umgebungsluft. Bevorzugt hat die Frischluft eine relative Feuchte von weniger als 10%.

### Bezugszeichenliste

- 1: Vorrichtung
- 2: erstes Ende
- 3: Anschluss
- 4: zweites Ende
- 5: unterer
Vorrichtungsabschnitt
- 6: Innengewindeloch
- 7: Innengewindeloch
- 8: Strömungskanal
- 9: Verdampferkörper
- 10: Ummantelung
- 11: oberer Vorrichtungsabschnitt
- 12: Verteilerkammer
- 13: Transportschraube
- 14: Reihe
- 15: Reihe
- 16: Sackloch
- 17: Einlassöffnung
- 18: Sacklochgrund
- 19: Bodenseite
- 20: Längsseite
- 21: Längsseite
- 22: Heizeinrichtung
- 23: Temperaturfühler
- 24: Bohrung
- 25: Befestigungsflansch
- 26: Gegenflansch
- 27: Anschluss
- 28: Zuführkanal
- 29: Luer-Lock-Anschluss
- 30: Aufnahme
- 31: Einstellschraube
- 32: Zylindermantelfläche
- 33: Anordnung
- 34: zu dekontaminierender Raum
- 35: Eingangsanschluss
- 36: Ausgangsanschluss
- 37: Filter
- 38: Frischluftleitung
- 39: Temperiereinrichtung
- 40: Leitung
- 41: Regulierleitung
- 42: Abführleitung
- 43: Filter
- 44: Bypassleitung
- 45: Deckelplatte
- 46: Versorgungskanäle
- 47: Verschlussplatte
- 48: Vertiefung
- 49: Heizstäbe
- 50: Öffnung
- 51: Kanten
- 52: Zuführung
- 53: Innengewindelöcher

## Patentansprüche

1. Vorrichtung zur Erzeugung von Dekontaminationsmitteldampf, insbesondere Wasserstoffperoxyddampf, umfassend einen Verdampferkörper (9), eine Heizeinrichtung (22) zum Erhitzen des Verdampferkörpers (9) sowie mehrere Zuführkanäle (28) zum Zuführen von zu verdampfender Dekontaminationsflüssigkeit, zu dem Verdampferkörper (9),
**dadurch gekennzeichnet,**
**dass** in dem, ein- oder mehrteiligen, Verdampferkörper (9) mehre Sacklöcher (16) vorgesehen sind, und dass den Sacklöchern (16) jeweils mindestens einer der Zuführkanäle (28) zugeordnet ist, und dass die Zuführkanäle derart angeordnet sind, dass das zu verdampfende Dekontaminationsmittel unmittelbar in die Sacklöcher (16) eintropfbar ist.

2. Vorrichtung nach Anspruch 1,
**dadurch gekennzeichnet, dass** die sich senkrecht zur Axialerstreckung der Sacklöcher (16) erstreckende Querschnittsfläche der Sacklöcher (16) zumindest axialabschnittsweise konstant ist.

3. Vorrichtung nach einem der Ansprüche 1 oder 2,
**dadurch gekennzeichnet, dass** die Querschnittsfläche der Sacklöcher (16) rechteckig konturiert ist.

4. Vorrichtung nach Anspruch 1,
**dadurch gekennzeichnet, dass** die sich senkrecht zur Axialerstreckung der Sacklöcher (16) erstreckende Querschnittsfläche der Sacklöcher (16) zumindest axialabschnittsweise, kontinuierlich mit zunehmendem Abstand von einer Einlassöffnung (17) abnimmt.

5. Vorrichtung nach einem der vorhergehenden Ansprüche,
**dadurch gekennzeichnet, dass** der Verdampferkörper (9) eine größere Längen- als Breitenerstreckung aufweist.

6. Vorrichtung nach einem der vorhergehenden Ansprüche,
**dadurch gekennzeichnet, dass** die Sacklöcher (16) in mindestens einer sich in Richtung der Längserstreckung des Verdampferkörpers (9) erstreckenden Reihe (14, 15) angeordnet sind.

7. Vorrichtung nach einem der vorhergehenden Ansprüche,
**dadurch gekennzeichnet, dass** die Heizeinrichtung (22) sich entlang zweier voneinander abgewandter Längsseiten (20, 21) des Verdampferkörpers (9) und/oder entlang einer unteren Bodenfläche und/oder entlang einer zwischen zwei Sacklochreihen (14, 15) angeordneten Zwischenebene, erstreckend angeordnet ist.

8. Vorrichtung nach einem der Ansprüche 1 bis 6,
**dadurch gekennzeichnet, dass** als Heizeinrichtung (22) mindestens ein Heizstab (49) vorgesehen ist, der in einer langgestreckten Öffnung (50) im Verdampferkörper (9) aufgenommen ist.

9. Vorrichtung nach einem der vorhergehenden Ansprüche,
**dadurch gekennzeichnet, dass** die Zuführkanäle (28) in die Sacklöcher (16) hineinragend angeordnet sind.

10. Vorrichtung nach einem der vorhergehenden Ansprüche,
**dadurch gekennzeichnet, dass** die Zuführkanäle (28) austauschbar ausgebildet und angeordnet sind.

11. Vorrichtung nach einem der vorhergehenden Ansprüche,
**dadurch gekennzeichnet, dass** die Zuführkanäle (28) von Injektionsnadeln gebildet sind.

12. Vorrichtung nach einem der vorhergehenden Ansprüche,
**dadurch gekennzeichnet, dass** den Zuführkanälen (28) eine über einen Strömungskanal (8) für Trägerluft von dem Verdampferkörper (9) beabstandete Verteilerkammer (12) zugeordnet ist.

13. Vorrichtung nach einem der vorhergehenden Ansprüche,
**dadurch gekennzeichnet, dass** jedem Zuführkanal (28) ein Versorgungskanal (46) zugeordnet ist, wobei die Versorgungskanäle (46) bevorzugt gleich lang sind und den gleichen Durchflussquerschnitt aufweisen und von einer gemeinsamen Versorgungsleitung versorgbar sind.

14. Anordnung, umfassend einen zu dekontaminierenden Raum (34), insbesondere einen Isolator und/oder eine Schleuse, sowie eine Vorrichtung (1) zur Erzeugung von Dekontaminationsmitteldampf, insbesondere Wasserstoffperoxyddampf, nach einem der vorhergehenden Ansprüche.

15. Anordnung nach Anspruch 14,
**dadurch gekennzeichnet, dass** die Vorrichtung (1), bevorzugt ausschließlich, mit Frischluft, insbesondere Druckluft oder Umgebungsluft, als Trägerluft von außerhalb des zu dekontaminierenden Raums (34) versorgbar angeordnet ist.

## Claims

1. Device for generating decontamination agent vapour, in particular hydrogen peroxide vapour, comprising an evaporator body (9), a heating unit (22) for heating the evaporator body (9) and a plurality of feed channels (28) for feeding decontamination liquid to be evaporated to the evaporator body (9), **characterised in that** a plurality of blind holes (16) is provided in the one-part or multi-part evaporator body (9) and **in that** the blind holes (16) are each assigned to at least one of the feed channels (28) and **in that** the feed channels are arranged in such a manner that the decontamination agent to be evaporated can be dropped directly into the blind holes (16).

2. Device according to claim 1, **characterised in that** the cross-sectional surface of the blind holes (16) extending perpendicular to the axial extension of the blind holes (16) is constant, at least in terms axial portions.

3. Device according to either claim 1 or claim 2, **characterised in that** the cross-sectional surface of the blind holes (16) has a rectangular contour.

4. Device according to claim 1, **characterised in that** the cross-sectional surface of the blind holes (16) extending perpendicular to the axial extension of the blind holes (16) diminishes continuously as the distance from the inlet opening (17) increases, at least in axial portions.

5. Device according to any of the preceding claims, **characterised in that** the evaporator body (9) has a length extension greater than the width extension thereof.

6. Device according to any of the preceding claims, **characterised in that** the blind holes (16) are disposed in at least one row (14, 15) extending in the direction of the longitudinal extension of the evaporator body (9).

7. Device according to any of the preceding claims, **characterised in that** the heating unit (22) is arranged extending along two longitudinal sides (20, 21) of the evaporator body (9) facing away from each other and/or along a lower base face and/or along an intermediate plane arranged between two rows (14, 15) of blind holes.

8. Device according to any of claims 1 to 6, **characterised in that** at least one heating rod (49) is provided as a heating unit (22), which heating rod is being accommodated in an elongate opening (50) in the evaporator body (9).

9. Device according to any of the preceding claims, **characterised in that** the feed channels (28) are arranged projecting into the blind holes (16).

10. Device according to any of the preceding claims, **characterised in that** the feed channels (28) are designed and arranged such that they are interchangeable.

11. Device according to any of the preceding claims, **characterised in that** the feed channels (28) are formed from injection needles.

12. Device according to any of the preceding claims, **characterised in that** a distributor chamber (12) spaced apart from the evaporator body (9) via a flow channel (8) for carrier air is assigned to the feed channels (28).

13. Device according to any of the preceding claims, **characterised in that** a supply channel (46) is assigned to each feed channel (28), the supply channels (46) preferably being the same length and having the same flow cross-section and being able to be supplied by a common supply line.

14. Assembly, comprising a space (34) to be decontaminated, in particular an isolator and/or a lock, and a device (1) for generating decontamination agent vapour, in particular hydrogen peroxide vapour, according to any of the preceding claims.

15. Assembly according to claim 14, **characterised in that** the device (1) is arranged such that it can be supplied, preferably exclusively, with fresh air, in particular compressed air or ambient air, as carrier air from outside the space (34) to be decontaminated.

## Revendications

1. Dispositif pour la production de vapeur d'agent de décontamination, en particulier de vapeur de peroxyde d'hydrogène, comprenant un corps d'évaporateur (9), un dispositif de chauffage (22) pour le chauffage du corps d'évaporateur (9) ainsi que plusieurs canaux d'arrivée (28) pour l'apport de liquide de décontamination à vaporiser au corps d'évaporateur (9), **caractérisé en ce qu'**il est prévu plusieurs trous borgnes (16) dans le corps d'évaporateur (9) en une ou plusieurs parties, et **en ce qu'**au moins un des canaux d'arrivée (28) est associé respectivement aux trous borgnes (16), et **en ce que** les canaux d'arrivée sont disposés de telle manière que l'agent de décontamination à vaporiser puisse goutter directement dans les trous borgnes (16).

2. Dispositif selon la revendication 1, **caractérisé en ce que** la surface de section transversale des trous borgnes (16) s'étendant perpendiculairement à l'extension axiale des trous borgnes (16) est constante au moins par tronçons axiaux.

3. Dispositif selon l'une quelconque des revendications 1 ou 2, **caractérisé en ce que** la surface de section transversale des trous borgnes (16) est de forme rectangulaire.

4. Dispositif selon la revendication 1, **caractérisé en ce que** la surface de section transversale des trous borgnes (16) s'étendant perpendiculairement à l'extension axiale des trous borgnes (16) diminue, au moins par tronçons axiaux, de façon continue avec l'augmentation de la distance à partir d'une ouverture d'entrée (17).

5. Dispositif selon l'une quelconque des revendications précédentes, **caractérisé en ce que** le corps d'évaporateur (9) présente une plus grande extension en longueur qu'en largeur.

6. Dispositif selon l'une quelconque des revendications précédentes, **caractérisé en ce que** les trous borgnes (16) sont disposés en au moins une rangée (14, 15) s'étendant dans la direction de l'extension en longueur du corps d'évaporateur (9).

7. Dispositif selon l'une quelconque des revendications précédentes, **caractérisé en ce que** le dispositif de chauffage (22) est disposé de façon à s'étendre le long de deux côtés longitudinaux opposés l'un à l'autre (20, 21) du corps d'évaporateur (9) et/ou le long d'une face de fond inférieure et/ou le long d'un plan intermédiaire disposé entre deux rangées de trous borgnes (14, 15).

8. Dispositif selon l'une quelconque des revendications 1 à 6, **caractérisé en ce qu'**il est prévu comme dispositif de chauffage (22) au moins un barreau chauffant (49), qui est logé dans une ouverture (50) s'étendant longitudinalement dans le corps d'évaporateur (9).

9. Dispositif selon l'une quelconque des revendications précédentes, **caractérisé en ce que** les canaux d'arrivée (28) sont disposés de façon à pénétrer dans les trous borgnes (16).

10. Dispositif selon l'une quelconque des revendications précédentes, **caractérisé en ce que** les canaux d'arrivée (28) sont réalisés et disposés de façon remplaçable.

11. Dispositif selon l'une quelconque des revendications précédentes, **caractérisé en ce que** les canaux d'arrivée (28) sont formés par des aiguilles d'injection.

12. Dispositif selon l'une quelconque des revendications précédentes, **caractérisé en ce qu'**une chambre de répartiteur (12) espacée du corps d'évaporateur (9) par un canal d'écoulement (8) pour l'air porteur est associée aux canaux d'arrivée (28).

13. Dispositif selon l'une quelconque des revendications précédentes, **caractérisé en ce qu'**un canal d'alimentation (46) est associé à chaque canal d'arrivée (28), dans lequel les canaux d'alimentation (46) ont de préférence la même longueur et présentent la même section mouillée et peuvent être alimentés par une conduite d'alimentation commune.

14. Agencement comprenant un espace à décontaminer (34), en particulier un isolateur et/ou un sas, ainsi qu'un dispositif (1) pour la production de vapeur d'agent de décontamination, en particulier de vapeur de peroxyde d'hydrogène, selon l'une quelconque des revendications précédentes.

15. Agencement selon la revendication 14, **caractérisé en ce que** le dispositif (1) est disposé de façon à pouvoir être alimenté, de préférence exclusivement, en air frais, en particulier en air comprimé ou en air atmosphérique, comme air porteur depuis l'extérieur de l'espace à décontaminer (34).
